# EUROPEAN PATENT SPECIFICATION

(11) **EP 3 471 732 B1**
(45) Date of publication and mention of the grant of the patent: **13.05.2020**
(21) Application number: 17731852.4
(22) Date of filing: 15.06.2017
(51) Int. Cl.: A61K 31/575, A61K 9/127, A61K 31/688, A61P 31/12

(54) **LIPOSOMES FOR THE TREATMENT OF VIRAL INFECTIONS**
LIPOSOME ZUR BEHANDLUNG VON VIRUSINFEKTIONEN
LIPOSOMES POUR LE TRAITEMENT D'INFECTIONS VIRALES

(30) Priority: 16.06.2016 EP 16174700
(43) Date of publication of application: 24.04.2019
(73) Proprietor: Combioxin SA, 1204 Geneva (CH)
(72) Inventor: AZEREDO DA SILVEIRA LAJAUNIAS, Samareh, 1206 Geneva (CH); LAJAUNIAS, Frédéric, 1206 Geneva (CH)
(74) Representative: Latscha Schöllhorn Partner AG
(86) International application number: PCT/EP2017/064653
(87) International publication number: WO 2017/216282

(56) References cited:
- WO-A1-2013/186286
- WO-A2-2007/135523
- DE HAAN A ET AL: "Mucosal immunoadjuvant activity of liposomes: induction of systemic IgG and secretory IgA responses in mice by intranasal immunization with an influenza subunit vaccine and coadministered liposomes", VACCINE, ELSEVIER, AMSTERDAM, NL, vol. 13, no. 2, 1 February 1995 (1995-02-01), pages 155-162, XP004057678, ISSN: 0264-410X, DOI: 10.1016/0264-410X(95)93129-W
- RAWAT SATINDER S ET AL: "Modulation of entry of enveloped viruses by cholesterol and sphingolipids (Review)", MOLECULAR MEMBRANE BIOLOGY, TAYLOR AND FRANCIS, GB, vol. 20, no. 3, 1 July 2003 (2003-07-01), pages 243-254, XP008150470, ISSN: 0968-7688, DOI: 10.1080/0968768031000104944
- HIDEKI AIZAKI ET AL: "Critical Role of Virion-Associated Cholesterol and Sphingolipid in Hepatitis C Virus Infection", JOURNAL OF VIROLOGY, THE AMERICAN SOCIETY FOR MICROBIOLOGY, US, vol. 82, no. 12, 1 June 2008 (2008-06-01), pages 5715-5724, XP008145731, ISSN: 0022-538X, DOI: 10.1128/JVI.02530-07 [retrieved on 2008-03-26]
- HAMPL J ET AL: "Enhancement of antibody response to bovine herpesvirus 1 with non-specific immunostimulants", VETERINARNI MEDICINA, CESKA AKADEMIE ZEMEDELSKYCH VED,CZECH ACADEMY OF AGRICULTURAL SCIENCES, CZ, vol. 41, no. 5, 1 May 1996 (1996-05-01), pages 143-147, XP008182339, ISSN: 0375-8427

## Description

The present invention relates to empty liposomes or mixtures of empty liposomes of defined lipid composition for use in the treatment and prevention of viral infections. The present invention further relates to compositions for use in the treatment of such viral infections comprising administering the inventive empty liposomes or mixtures of empty liposomes, alone or in combination with standard viral treatment.

### RELATED ART

Viral infections are an important public health concern and burden worldwide. As a consequence, a major focus is on understanding the compositions and mechanisms underlying the fusion with and entry into the host cells by the viruses.

Cell membrane lipid rafts have been shown to be involved in the virus fusion, entry, assembly, or/and budding process in infection lifecycles of various viruses such as retroviruses (*Retroviridae*), RNA viruses (*Arenaviridae, Astroviridae, Bunyaviridae, Caliciviridae, Coronaviridae, Filoviridae, Flaviviridae, Orthomyxoviridae, Paramyxoviridae, Picornaviridae, Reoviridae, Rhabdoviridae,* and *Togaviridae*), and DNA viruses (*Adenoviridae, Hepadnaviridae, Herpesviridae, Papovaviridae, Parvoviridae,* and *Poxviridae*). These studies have demonstrated the localization of viral structural proteins in lipid rafts and the effects of raft-disrupting agents, which mainly remove cholesterol from the surface membrane or inhibit the synthesis of cholesterol, on the infection and replication processes of these viruses (Takahashi, T and Suzuki, T Biochem Res Intern 2011; Article ID 245090).

Cell membrane lipid rafts that are highly enriched in cholesterol and sphingomyelin appear to act as a platform for viral fusion and entry, in particular, of many enveloped viruses into target cells. Studies propose that during the fusion process, viruses hijack host rafts for cell entry by causing a concentration of virus receptors, altering the conformation of virus receptors, allowing to traffic to the appropriate cell compartment, and/or mediating conformational changes in the virus envelop (Manes, S et al. Nat Rev Immunol 2003; 3:557-568; Rawat SS et al. Mol Membr Biol 2003; 20:243-254).

Enveloped viruses believed to display said mode of viral infection include those of the following families, namely *Herpesviridae* (Herpes simplex virus, varicella-zoster virus, cytomegalovirus, Epstein-Barr virus), *Hepadnaviridae* (Hepatitis B virus), *Togaviridae* (Rubella virus, alphavirus), *Arenaviridae* (lymphocytic choriomeningitis virus), *Flaviviridae* (Dengue virus, hepatitis C virus, yellow fever), *Orthomyxoviridae* (Influenzavirus A, influenzavirus B, influenzavirus C, isavirus, thogotovirus), *Paramyxoviridae* (Measles virus, mumps virus, respiratory syncytial virus, Rinderpest virus, canine distemper virus), *Bunyaviridae* (California encephalitis virus, hantavirus), *Rhabdoviridae* (Rabies virus), *Filoviridae* (Ebola virus, Marburg virus), *Coronaviridae* (Corona virus), *Bornaviridae,* (Borna disease virus), *Arteriviridae* (Arterivirus, equine arteritis virus) and *Retroviridae* (Human immunodeficiency virus).

In particular and of major concern, the Hepatitis C virus (HCV), an enveloped virus belonging to the genus *Hepacivirus* of the *Flaviviridae* family, has infected some 170 million people worldwide. In the majority of HCV-infected patients, the progression to chronic disease is associated with an increased risk of liver diseases and hepatocellular carcinoma. The precise mechanisms regulating HCV fusion and entry into hepatic cells are still unknown and factors cooperating to complete the fusion and entry processes are not yet concertedly defined. It has been suggested that cholesterol and sphingolipid associated with hepatitis C virus (HCV) particles are important for virion maturation and infectivity and that depletion of cholesterol from the virus or hydrolysis of virion-associated sphingomyelin almost completely abolished HCV infectivity (Aizaki, H. et al., Journal of Virology 2008; 82:5715-5724). A reagent removing cellular cholesterol was shown to have an inhibitory effect on HCV entry through the virus receptor CD81 within lipid rafts (Kapadia B et al. J Virol 2007; 81(1): 374-383). Cellular sphingomyelin hydrolysis is also believed to show a strong inhibitory effect on HCV entry. This was suggested to be due to the fact that ceramide enrichment of the plasma membrane leads to a decreased level of CD81 at the cell surface membrane by enhancement of CD81 internalization (Voisset C et al. Cellular Microbiology 2008; 10(3): 606-617). Liposomes have been used in studies aiming at better understanding HCV fusion and infection processes. For example, studies using liposomes composed of phosphatidylcholine or phosphatidylcholine:cholesterol (70:30 molar ratio) proposed that, although fusion did not strictly rely on the presence of cholesterol, it was facilitated by the presence of cholesterol in the target membrane (Lavillette D et al. J Biol Chem 2006; 281(7):3909-3917). Furthermore, studies using phosphatidylcholine, phosphatidylcholine:cholesterol (70:30 molar ratio), phosphatidylcholine:sphingomyelin (90:10 molar ratio) or phosphatidylcholine:cholesterol:sphingomyelin (65:30:5 molar ratio) showed that HCV fusion is not only facilitated by cholesterol but further enhanced when a combination between sphingomyelin and cholesterol is present in the target membranes, and propose that cholesterol and sphingomyelin could act in synergy to convey optimal HCV fusion (Haid, s et al., J Biol Chem. 2009; 284(26):17657-17667).

The importance of cholesterol-rich lipid rafts has further been reported for human immunodeficiency virus (HIV) pathogenesis. It has been shown that HIV budding occurs at said cholesterol-rich lipid rafts and that host cell cholesterol is required for HIV infection. Moreover, it has been reported that removal of cholesterol from the membrane of HIV-infected cells dramatically lowered virus release and that virions released from cholesterol-depleted cells are minimally infectious (Liao Z et al. Aids Res Hum Retroviruses 2003; 19(8):675-687 and references cited therein). It has also been proposed that HIV fusion peptide preferentially targets T cell membrane raft domains and that these domains are necessary and sufficient for efficient membrane targeting and fusion (Yang et al. Nat Chem Biol 2015, DOI:10.1038).

The importance of cholesterol-rich lipid rafts has also been reported for herpes simplex virus type-1 (HSV-1) infections which cause significant health problems, including skin and corneal lesions and encephalitis. HSV appears to enter cells by direct fusion and lipids rafts have been identified as domains with highly fusogenic activity, and cholesterol within these rafts as a key player for HSV-1 fusion to cells (Rahn E et al. PlosOne 2011 6(10): e25464; Bender FC et al. J Virol 2003; 77(17):9542-9552; Vitiello G et al. Soft Matter. 2015; 11(15):3003-3016).

Lipid raft microdomains enriched in sphingomyelin and cholesterol also function as platforms for influenza-cell attachment and membrane fusion. A recent study using liposomes of various phospholipidic composition including cholesterol amounts up to 40% in molar ratio, reports that cholesterol acts in a dose-dependent manner to promote influenza fusion efficiency (Domanska, MK et al. Biophys J 2013; 105:1383-1387). Moreover, the effects on influenza virus fusion have been shown to be related to the packing characteristics of the phospholipid headgroups in the target membrane (Herrmann A et al. Membr Biochem 1993). Both Ebola virus (EboV) and Marburg virus (MbgV) are enveloped, single negative-stranded RNA viruses belonging to the family *Filoviridae.* Ebola virus uses plasma membrane lipid raft microdomains enriched in cholesterol and sphingomyelin for entry, assembly and budding. Cholesterol removing reagents or cholesterol synthesis inhibitors or depletion of sphingomyelin within cell membranes have inhibitory effects on Ebola viral infectivity. Moreover, it has been reported that the initial step of Ebola virus infection depends on cholesterol in targeted lipid rafts and that Ebola virus particles associate with the sphingomyelin-rich regions of the cell membrane (Miller et al. J Virol 2012; 86(14):7473-7483; Freitas MS et al. PLoS One. 2011; 6(1):e15756; and references cited therein).

Several studies suggest that the integrity of plasma membrane cholesterol- and sphingolipid-enriched lipid rafts is important for herpesviruses entry. Notably, lipid rafts were suggested as being essential for Varicella-Zoster virus (VZV, one of eight herpesviruses, also known as chickenpox virus or human herpesvirus type 3 (HHV-3)), which causes chickenpox in children, teens and young adults and herpes zoster in adults (Hambleton et al. J Virol 2007; 81(14):7548-7558) and for pseudorabies virus entry (Desplanques AS et al. Virology 2008; 376:339-345).

Measles virus remains a highly contagious disease and a substantial cause of morbidity and mortality. Glycosphingolipid-cholesterol-enriched membrane microdomains play an important role in the virus ability to suppress immune responses. Measles virus directly interacts with cholesterol-enriched lipid rafts at the surface of T cells, thereby recruiting and activating membrane proximal signaling components leading to a pronounced inhibition of T-cell proliferation (Avota et al. J Virol 2004; 78(17):9552-9559).

Human herpesvirus 6 (HHV-6) is a human pathogen of emerging clinical significance. Lipid rafts and intact cholesterol in the cellular membrane play an important role in the HH6 viral entry process. More precisely, HHV-6 was suggested to enter its target cells through a lipid raft and HHV-6 cellular receptor (CD46) to accumulate in lipid rafts after virus attachment, being thus recruited and then distributed in the lipid rafts for viral entry (Tang et al. Virol 2008; 378:265-271).

Tailored empty liposomes composed of cholesterol (CHOL) and sphingomyelin (SM) and their use for the treatment of bacterial infections have recently been described (WO 2013/186286; Henry BD et al., Nat Biotechnol 2015; 33(1):81-88; Azeredo da Silveira, S and Perez, A, Expert Rev. Anti Infect. Ther. 2015; 13(5):531-533). WO2007135523 discloses non-phospholipid Lipid Vesicle (nPLV) that interacts with an enveloped virus, and an agent that enhances lipid exchange between nPLV and enveloped virus, useful for treating enveloped virus-associated diseases.

### SUMMARY OF THE INVENTION

We have surprisingly found that empty liposomes of defined lipid composition and the mixtures of empty liposomes of defined lipid composition in accordance with the present invention display antiviral efficacy as shown in *in vitro* studies using pseudo-typed HCV particles. Thus, the empty liposomes and mixtures of empty liposomes of defined lipid composition are able to protect against viral infections, in particular, against infections caused by enveloped virus. The empty liposomes and mixtures of empty liposomes in accordance with the present invention are particularly able to protect against viral infections in a dose-dependent manner at the entry step of the virus, such as HCV, into the host cell. Notably, in the presence of the empty liposomes and mixtures of empty liposomes of defined lipid composition, entry of pseudo-typed HCV particles into target human hepatocyte cells is significantly decreased as measured by luciferase activity reflecting the degree of entry of the pseudo-particles into the cells.

Without being bound by this explanation, it is believed that the empty liposomes and mixtures of empty liposomes in accordance with the present invention mimic raft microdomains recognized by several viruses and are able to act as competitors for cellular lipid rafts in binding the viruses. Moreover, it is believed that the empty liposomes and mixtures of empty liposomes in accordance with the present invention act as a decoy to which, in particular, the viral envelope binds. Due to this believed mode of action and the shared attraction to and dependence of specific cellular lipid rafts, in particular CHOL and SM-composed microdomains, for the early stages of cell infection by the viruses, the empty liposomes and mixtures of empty liposomes in accordance with the present invention are able to display anti-viral activity for a wide variety of viruses. Furthermore, the empty liposomes in accordance with the present invention are considered non-toxic and allow for much higher relative concentrations of particular lipids than those ever likely to occur *in vivo.*

Thus, in first aspect, the present invention provides for a composition for use in a method of treating or preventing, preferably of treating, a viral infection in a mammal, preferably in a human, wherein said composition comprises, preferably consists of, (i) a single empty liposome, wherein said single empty liposome is selected from (a) an empty liposome consisting of sphingomyelin and cholesterol, wherein the amount of cholesterol is at least 20% (weight per weight); or (b) an empty liposome consisting of sphingomyelin; or (ii) a mixture of empty liposomes, wherein said mixture of empty liposomes comprises (a) a first empty liposome consisting of sphingomyelin and cholesterol, wherein the amount of cholesterol is at least 20% (weight per weight), and (b) a second empty liposome consisting of sphingomyelin.

In a further aspect, the present invention provides for an empty liposome consisting of sphingomyelin and cholesterol, wherein the amount of cholesterol is at least 20% (weight per weight) for use in a method of treating or preventing, preferably of treating, a viral infection in a mammal, preferably in a human.

In a further aspect, the present invention provides for an empty liposome consisting of sphingomyelin for use in a method of treating or preventing, preferably of treating, a viral infection in a mammal, preferably in a human.

In still another aspect, the present invention provides for a mixture of empty liposomes comprising (a) first empty liposome consisting of sphingomyelin and cholesterol, wherein the amount of cholesterol is at least 20% (weight per weight); an (b) a second empty liposome consisting of sphingomyelin; for use in a method of treating or preventing, preferably of treating, a viral infection in a mammal, preferably in a human.

In still another aspect, the present invention provides for compositions for use in a treatment of a viral infection comprising administering to a mammal, preferably a human, in need thereof a therapeutically effective amount of the inventive composition, the inventive single empty liposomes or the inventive mixture of empty liposomes, and to a method of prevention of a viral infection in a subject at risk. In yet another aspect, the present invention provides for a treatment or prevention of a viral infection comprising administering to a mammal, preferably a human, in need thereof a therapeutically effective amount of the inventive composition, the inventive single empty liposomes or the inventive mixture of empty liposomes without coadministering a standard antiviral treatment. Furthermore the invention relates to a treatment of viral infections comprising administering to a mammal, preferably a human, in need thereof a therapeutically effective amount of the inventive composition, the inventive single empty liposomes or the inventive mixture of empty liposomes, before, after, together or in parallel with a standard antiviral treatment of the viral infection.

Further aspects and embodiments of the present invention will become apparent as this description continues.

### DESCRIPTION OF FIGURES

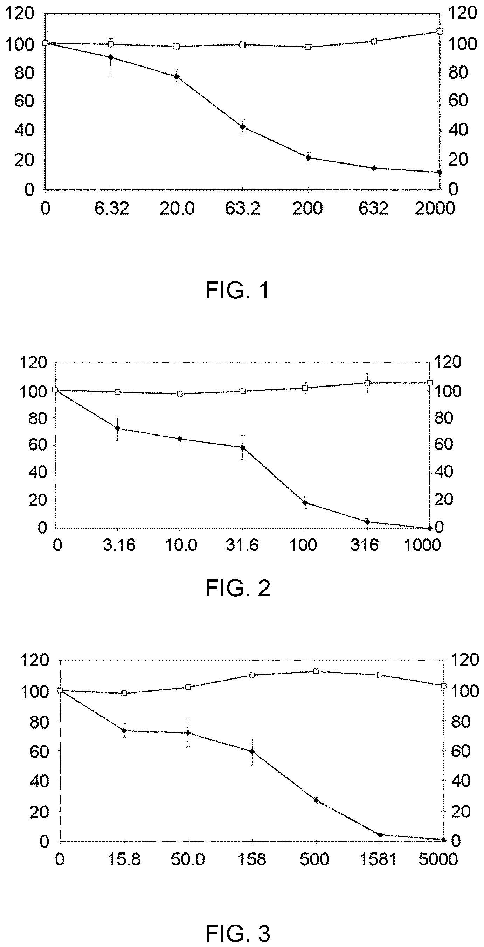

- FIG. 1:: Inhibition of HCV-1b E1E2 pseudotype virus by an inventive mixture of empty liposomes: Dose-dependent inhibition of HCV entry step into Huh7.5 cells by various concentrations of said inventive mixture of empty liposomes (using 6 dilutions, from 0 to 2000 ug/ml). Left scale and black lozenges indicate the percentage of virus entry inhibition relative to control infections lacking said inventive mixture of empty liposomes (%VC). Right scale and white squares indicate the percentage of cytotoxicity as relative to cell death of Huh7.5 cells not treated with said inventive mixture of empty liposomes (%CC).
- FIG. 2:: Inhibition of HCV-1b E1E2 pseudotype virus by cyanovirin (PG51) as a positive control. Dose-dependent inhibition of HCV entry step into Huh7.5 cells by various concentrations of cyanovirin (using 6 dilutions, from 0 to 1000 ug/ml). Left scale and black lozenges indicate the percentage of virus entry inhibition relative to control infections lacking cyanovirin (%VC). Right scale and white squares indicate the percentage of cytotoxicity as relative to cell death of Huh7.5 cells not treated with cyanovirin (%CC).
- FIG. 3:: Inhibition of HCV-1b E1E2 pseudotype virus by anti-CD81 as a positive control. Dose-dependent inhibition of HCV entry step into Huh7.5 cells by various concentrations of anti-CD81 (using 6 dilutions, from 0 to 1000 ug/ml). Left scale and black lozenges indicate the percentage of virus entry inhibition relative to control infections lacking anti-CD81 (%VC). Right scale and white squares indicate the percentage of cytotoxicity as relative to cell death of Huh7.5 cells not treated with anti-CD81 (%CC).

### DETAILED DESCRIPTION OF THE INVENTION

Unless defined otherwise, all technical and scientific terms used herein have the same meanings as commonly understood by one of ordinary skill in the art to which this invention belongs.

The term "about", as used herein shall have the meaning of +/- 10%. For example about 50% shall mean 45% to 55%. Preferably, the term "about", as used herein shall have the meaning of +/- 5%. For example about 50% shall mean 47.5% to 52.5%.

When the terms "a," or "an" are used herein, they mean "at least one" unless indicated otherwise. In particular, the use of the terms "a," or "an" in association with said single empty liposome, said first empty liposome and said second empty liposome to describe the empty liposomes and mixtures of empty liposomes in accordance with the present invention should typically and preferably refer to single empty liposomes and mixtures of empty liposomes comprising said first empty liposomes and said second empty liposomes.

Thus, in first aspect, the present invention provides for a composition for use in a method of treating or preventing, preferably of treating, a viral infection in a mammal, preferably in a human, wherein said composition comprises, preferably consists of, (i) a single empty liposome, wherein said single empty liposome is selected from (a) an empty liposome consisting of sphingomyelin and cholesterol, wherein the amount of cholesterol is at least 20% (weight per weight); or (b) an empty liposome consisting of sphingomyelin; or (ii) a mixture of empty liposomes, wherein said mixture of empty liposomes comprises (a) a first empty liposome consisting of sphingomyelin and cholesterol, wherein the amount of cholesterol is at least 20% (weight per weight), and (b) a second empty liposome consisting of sphingomyelin.

The term "empty liposome" as used herein, refers to liposomes, preferably artificial liposomes, having a mean diameter of 20 nm to 10 µm, preferably of 20 to 500 nm, and further preferably having a mean diameter of 20 nm to 200 nm, and consist of one or more phospholipid bilayers, and are typically and preferably unilamellar vesicles and multilamellar vesicles, more preferably small unilamellar vesicles (SUVs). In a preferred embodiment, the term "empty liposome" as used herein, typically and preferably refers to liposomes not incorporating any drug, typically and preferably to liposomes not incorporating any pharmaceutical drug. "Incorporated/Incorporating" as used herein and when referring to the inventive empty liposomes, typically and preferably, means encapsulated/encapsulating into the cavity of the liposome, within the potential double layer of the liposome, or as part of the membrane layer of the liposome. In another preferred embodiment, the term "empty liposome" as used herein, typically and preferably refers to liposomes consisting of sphingomyelin and cholesterol or consisting of sphingomyelin in accordance with the present invention and solely further comprises water-soluble inorganic compounds and/or water-soluble organic molecules, wherein typically and preferably said water-soluble inorganic compounds and/or water-soluble organic molecules derive from the synthesis of said inventive empty liposomes, and wherein typically and preferably, said water-soluble inorganic compounds are inorganic salts preferably selected from NaCl, KCl, MgCl₂, and wherein said water-soluble organic molecules are buffering agents, wherein preferably said water-soluble organic molecules are selected from glucose and HEPES. Typically and preferably, said water-soluble inorganic compounds and/or water-soluble organic molecules are incorporated in the inventive empty liposomes of the present invention due to their presence during the production of the inventive empty liposomes. In another preferred embodiment, the term "empty liposome" as used herein, typically and preferably refers to liposomes consisting of sphingomyelin and cholesterol or consisting of sphingomyelin in accordance with the present invention and wherein said empty liposomes do not comprise an antioxidant. In a further preferred embodiment, the term "empty liposome" as used herein, typically and preferably refers to liposomes consisting of sphingomyelin and cholesterol or consisting of sphingomyelin in accordance with the present invention and solely further comprises water-soluble inorganic compounds and/or water-soluble organic molecules, wherein typically and preferably said water-soluble inorganic compounds and/or water-soluble organic molecules derive from the synthesis of said inventive empty liposomes, and wherein typically and preferably, said water-soluble inorganic compounds are inorganic salts preferably selected from NaCl, KCl, MgCl₂, and wherein said water-soluble organic molecules are buffering agents, wherein preferably said water-soluble organic molecules are selected from glucose and HEPES, and wherein said empty liposomes consisting of sphingomyelin and cholesterol or consisting of sphingomyelin in accordance with the present invention do not comprise an antioxidant.

In a preferred embodiment, said composition for use in the method in accordance with the invention comprises, preferably consists of, a single empty liposome, wherein said single empty liposome is selected from (a) an empty liposome consisting of sphingomyelin and cholesterol, wherein the amount of cholesterol is at least 20% (weight per weight); or (b) an empty liposome consisting of sphingomyelin.

In another preferred embodiment, said composition for use in said method in accordance with the invention comprises, preferably consists of, a single empty liposome, wherein said single empty liposome is an empty liposome consisting of sphingomyelin and cholesterol, wherein the amount of cholesterol is at least 20% (weight per weight). Preferably, the amount of cholesterol of said empty liposome is 20%-70% (weight per weight), further preferably the amount of cholesterol of said empty liposome is 25%-60% (weight per weight). In another preferred embodiment, the amount of cholesterol of said empty liposome is 45%-55% (weight per weight), and again further preferably said amount of cholesterol of said empty liposome is about 50% (weight per weight). Thus, in a very preferred embodiment of the present invention, said single empty liposome comprises, preferably consists of 50% (weight per weight) sphingomyelin and of 50% (weight per weight) cholesterol for use in a method of treating a viral infection in a mammal, preferably in a human.

In another preferred embodiment, said composition for use in said method in accordance with the invention comprises, preferably consists of, a single empty liposome, wherein said single empty liposome is an empty liposome consisting of sphingomyelin.

In another preferred embodiment, said composition for use in said method in accordance with the invention comprises, preferably consists of a mixture of empty liposomes, wherein said mixture of empty liposomes comprises (a) a first empty liposome consisting of sphingomyelin and cholesterol, wherein the amount of cholesterol is at least 20% (weight per weight); and (b) a second empty liposome consisting of sphingomyelin. Preferably, the amount of cholesterol of said first empty liposome is 20%-70% (weight per weight), further preferably the amount of cholesterol of said first empty liposome is 25%-60% (weight per weight). In another preferred embodiment, the amount of cholesterol of said first empty liposome is 45%-55% (weight per weight), and again further preferably said amount of cholesterol of said first empty liposome is about 50% (weight per weight). Thus, in a very preferred embodiment of the present invention, said first empty liposome comprises, preferably consists of 50% (weight per weight) sphingomyelin and of 50% (weight per weight) cholesterol for use in a method of treating a viral infection in a mammal, preferably in a human.

In another preferred embodiment, said mixture of empty liposomes comprises at least 30% (weight per weight) of said first empty liposome and at most 70% (weight per weight) of said second empty liposome, and wherein preferably said mixture of empty liposomes comprises at least 40% (weight per weight) of said first empty liposome and at most 60% (weight per weight) of said second empty liposome. In a further preferred embodiment, said mixture of empty liposomes comprises at least 45% (weight per weight) of said first empty liposome and at most 55% (weight per weight) of said second empty liposome, and wherein preferably said mixture of empty liposomes comprises about 50% (weight per weight) of said first empty liposome and about 50% (weight per weight) of said second empty liposome.

In another preferred embodiment, said mixture of empty liposomes consists of said first empty liposome and said second empty liposome.

In a further preferred embodiment, said mixture of empty liposomes consists of said first empty liposome and said second empty liposome, and wherein said mixture of empty liposomes consists of at least 40% (weight per weight) of said first empty liposome and at most 60% (weight per weight) of said second empty liposome, and wherein preferably said mixture of empty liposomes consists of about 50% (weight per weight) of said first empty liposome and about 50% (weight per weight) of said second empty liposome.

In a further preferred embodiment, said mixture of empty liposomes consists of said first empty liposome and said second empty liposome, and wherein said mixture of empty liposomes consists of at least 40% (weight per weight) of said first empty liposome and at most 60% (weight per weight) of said second empty liposome, and wherein preferably said mixture of empty liposomes consists of about 50% (weight per weight) of said first empty liposome and about 50% (weight per weight) of said second empty liposome, and wherein the amount of cholesterol of said first empty liposome is 45%-55% (weight per weight), and wherein preferably said the amount of cholesterol of said first empty liposome is about 50% (weight per weight).

In a further preferred embodiment, the empty liposomes used or for use in the present invention, are liposomes, preferably artificial liposomes, having a mean diameter of 20 nm to 10 µm, preferably of 20 to 500 nm, and further preferably having a mean diameter of 20 nm to 200 nm. Empty liposomes used or for use in the present invention are liposomes not encapsulating any drug. Empty liposomes used or for use in the present invention do not comprise any drug. Empty liposomes used or for use in the present invention do not incorporate other drugs. "Incorporated" as used herein, typically and preferably means encapsulated into the cavity of the liposome, within the potential double layer of the liposome, or as part of the membrane layer of the liposome. The liposomes used or for use in the present invention consist of one or more phospholipid bilayers, typically and preferably unilamellar and multilamellar vesicles. Most preferred are small unilamellar vesicles (SUVs).

In a further aspect, the present invention provides for an empty liposome consisting of sphingomyelin and cholesterol, wherein the amount of cholesterol is at least 20% (weight per weight) for use in a method of treating or preventing, preferably of treating, a viral infection in a mammal, preferably in a human. In another preferred embodiment, said empty liposome for use in said method in accordance with the invention comprises, preferably consists of, sphingomyelin and cholesterol, wherein the amount of cholesterol is 20%-70% (weight per weight), further preferably the amount of cholesterol of said empty liposome is 25%-60% (weight per weight). In another preferred embodiment, the amount of cholesterol of said empty liposome is 45%-55% (weight per weight), and again further preferably the amount of cholesterol of said empty liposome is about 50% (weight per weight). Thus, in a very preferred embodiment of the present invention, said empty liposome comprises, preferably consists of 50% (weight per weight) sphingomyelin and of 50% (weight per weight) cholesterol for use in a method of treating a viral infection in a mammal, preferably in a human.

In a further aspect, the present invention provides for an empty liposome consisting of sphingomyelin for use in a method of treating or preventing, preferably of treating, a viral infection in a mammal, preferably in a human.

In still another aspect, the present invention provides for a mixture of empty liposomes comprising (a) first empty liposome consisting of sphingomyelin and cholesterol, wherein the amount of cholesterol is at least 20% (weight per weight); an (b) a second empty liposome consisting of sphingomyelin; for use in a method of treating or preventing, preferably of treating, a viral infection in a mammal, preferably in a human. Preferably, the amount of cholesterol of said first empty liposome is 20%-70% (weight per weight), further preferably the amount of cholesterol of said first empty liposome is 25%-60% (weight per weight). In another preferred embodiment, the amount of cholesterol of said first empty liposome is 45%-55% (weight per weight), and again further preferably said amount of cholesterol of said first empty liposome is about 50% (weight per weight). Thus, in a very preferred embodiment of the present invention, said first empty liposome comprises, preferably consists of 50% (weight per weight) sphingomyelin and of 50% (weight per weight) cholesterol for use in a method of treating a viral infection in a mammal, preferably in a human. In another preferred embodiment, said mixture of empty liposomes comprises at least 30% (weight per weight) of said first empty liposome and at most 70% (weight per weight) of said second empty liposome, and wherein preferably said mixture of empty liposomes comprises at least 40% (weight per weight) of said first empty liposome and at most 60% (weight per weight) of said second empty liposome. In a further preferred embodiment, said mixture of empty liposomes comprises at least 45% (weight per weight) of said first empty liposome and at most 55% (weight per weight) of said second empty liposome, and wherein preferably said mixture of empty liposomes comprises about 50% (weight per weight) of said first empty liposome and about 50% (weight per weight) of said second empty liposome.

Thus, in a very preferred embodiment of the present invention, said mixture of empty liposomes comprises, preferably consists of, (i) a first empty liposome consisting of about 50% (weight per weight) sphingomyelin and of about 50% (weight per weight) cholesterol, and (ii) a second empty liposome consisting of (100%) sphingomyelin; for use in a method of treating or preventing a viral infection.

The liposomes are manufactured according to extrusion or sonication or micro fluidization (e.g. high pressure homogenization) methods known in the art. For example, the lipids are mixed in an organic solvent such as chloroform. Chloroform is evaporated and the dry lipid film is hydrated in an aqueous solution such as normal saline (0.9% NaCl), Krebs solution, or Tyrode's solution and further sonicated to produce liposomes. If necessary, the size of the liposomes can be controlled by their extrusion through membrane filters of fixed pore diameter. Individually produced liposomes of different lipid compositions are mixed in the required proportions typically and preferably just before application.

The lipid surface (bilayer) of liposomes forms spontaneously in water-based solvents and therefore traps water and other water-soluble inorganic and organic molecules, which might be present during liposome production, inside the liposome. The empty liposomes, used in the present study, are liposomes produced in buffers containing water and simple organic or inorganic molecules (for example NaCl, KCl, MgCl₂, glucose, HEPES, and/or CaCl₂).

In another preferred embodiment of the present invention, said viral infection is an infection by an enveloped virus. In a further preferred embodiment of the present invention, said viral infection is an infection by an enveloped virus, and wherein said enveloped virus is selected from herpes simplex virus, varicella-zoster virus, cytomegalovirus, Epstein-Barr virus, hepatitis B virus, rubella virus, alphavirus, lymphocytic choriomeningitis virus, dengue virus, hepatitis C virus, yellow fever, influenzavirus A, influenzavirus B, influenzavirus C, isavirus, thogotovirus, measles virus, mumps virus, respiratory syncytial virus, rinderpest virus, canine distemper virus, california encephalitis virus, hantavirus, rabies virus, Ebola virus, Marburg virus, corona virus, borna disease virus, arterivirus, equine arteritis virus and human immunodeficiency virus. Further preferably, said enveloped virus is selected from a hepatitis C virus (HCV), an immunodeficiency virus (HIV), a T lymphotrophic virus, a herpesvirus, a measles virus, a chicken pox virus, an influenza virus, an Ebola virus or a Marburg virus, again further preferably said enveloped virus is a hepatitis C virus (HCV). In a further preferred embodiment of the present invention, said viral infection is an infection by an enveloped virus, and wherein said enveloped virus is selected from a hepatitis C virus (HCV), an immunodeficiency virus (HIV), a T lymphotrophic virus, a herpesvirus, a measles virus, a chicken pox virus, an influenza virus, an Ebola virus or a Marburg virus. In a further preferred embodiment of the present invention, said viral infection is an infection by an enveloped virus, and wherein said enveloped virus is selected from a hepatitis C virus (HCV), an immunodeficiency virus (HIV), a T lymphotrophic virus, a herpesvirus, a measles virus, a chicken pox virus, an Ebola virus or a Marburg virus.

In another preferred embodiment of the present invention, said viral infection is an infection by a virus selected from herpes simplex virus, varicella-zoster virus, cytomegalovirus, Epstein-Barr virus, hepatitis B virus, rubella virus, alphavirus, lymphocytic choriomeningitis virus, dengue virus, hepatitis C virus, yellow fever, influenzavirus A, influenzavirus B, influenzavirus C, isavirus, thogotovirus, measles virus, mumps virus, respiratory syncytial virus, rinderpest virus, canine distemper virus, california encephalitis virus, hantavirus, rabies virus, Ebola virus, Marburg virus, corona virus, borna disease virus, arterivirus, equine arteritis virus and human immunodeficiency virus.

In another preferred embodiment of the present invention, said viral infection is an infection by a virus selected from a hepatitis C virus (HCV), an immunodeficiency virus (HIV), a T lymphotrophic virus, a herpesvirus, a measles virus, a chicken pox virus, an influenza virus, an Ebola virus or a Marburg virus.

In another preferred embodiment of the present invention, said viral infection is an infection by a virus selected from a hepatitis C virus (HCV), an immunodeficiency virus (HIV), a T lymphotrophic virus, a herpesvirus, a measles virus, a chicken pox virus, an Ebola virus or a Marburg virus.

In another very preferred embodiment of the present invention, said viral infection is an infection by an enveloped virus, wherein said enveloped virus is a hepatitis C virus.

In another very preferred embodiment of the present invention, said viral infection is an infection by a hepatitis C virus.

In a very preferred embodiment of the present invention, the composition comprises, preferably consist of, a mixture of empty liposomes comprising (a) first empty liposome consisting of sphingomyelin and cholesterol, wherein the amount of cholesterol is about 50% (weight per weight); an (b) a second empty liposome consisting of sphingomyelin; for use in a method of treating a viral infection in a mammal, preferably in a human, wherein said viral infection is an infection by a hepatitis C virus. Preferably, said mixture of empty liposomes comprises at least 40% (weight per weight) of said first empty liposome and at most 60% (weight per weight) of said second empty liposome. More preferably, said mixture of empty liposomes comprises about 50% (weight per weight) of said first empty liposome and about 50% (weight per weight) of said second empty liposome.

In another very preferred embodiment of the present invention, said viral infection is an infection by an enveloped virus, wherein said enveloped virus is a herpesvirus.

In another very preferred embodiment of the present invention, said viral infection is an infection by a herpesvirus.

In another very preferred embodiment of the present invention, said viral infection is an infection by an enveloped virus, wherein said enveloped virus is a herpesvirus, wherein said herpesvirus is a herpes simplex virus type-1 (HSV-1).

In another very preferred embodiment of the present invention, said viral infection is an infection by a herpesvirus, wherein said herpesvirus is a herpes simplex virus type-1 (HSV-1).

In another very preferred embodiment of the present invention, said viral infection is an infection by an enveloped virus, wherein said enveloped virus is a herpesvirus, wherein said herpesvirus is a human herpesvirus 6 (HHV6).

In another very preferred embodiment of the present invention, said viral infection is an infection by a herpesvirus, wherein said herpesvirus is a human herpesvirus 6 (HHV6).

In another very preferred embodiment of the present invention, said viral infection is an infection by an enveloped virus, wherein said enveloped virus is an immunodeficiency virus.

In another very preferred embodiment of the present invention, said viral infection is an infection by an immunodeficiency virus.

In another very preferred embodiment of the present invention, said viral infection is an infection by an enveloped virus, wherein said enveloped virus is an immunodeficiency virus, wherein said an immunodeficiency virus is an immunodeficiency virus type 1 (HIV-1).

In another very preferred embodiment of the present invention, said viral infection is an infection by an immunodeficiency virus, wherein said an immunodeficiency virus is an immunodeficiency virus type 1 (HIV-1).

In another very preferred embodiment of the present invention, said viral infection is an infection by an enveloped virus, wherein said enveloped virus is an influenza virus.

In another very preferred embodiment of the present invention, said viral infection is an infection by an influenza virus.

In another very preferred embodiment of the present invention, said viral infection is an infection by an enveloped virus, wherein said enveloped virus is a measles virus.

In another very preferred embodiment of the present invention, said viral infection is an infection by a measles virus.

In another very preferred embodiment of the present invention, said viral infection is an infection by an enveloped virus, wherein said enveloped virus is an Ebola virus.

In another very preferred embodiment of the present invention, said viral infection is an infection by an Ebola virus.

In another very preferred embodiment of the present invention, said viral infection is an infection by an enveloped virus, wherein said enveloped virus is a Marburg virus.

In another very preferred embodiment of the present invention, said viral infection is an infection by a Marburg virus.

In further embodiments, the method of treating or prevention a viral infection in accordance with the present invention further comprises the administration of a therapeutically effective amount of the inventive composition, the inventive single empty liposomes or the inventive mixture of empty liposomes to a mammal, preferably in a human in need thereof.

For the treatment of viral infections, the inventive single empty liposomes or the inventive mixture of empty liposomes are applied in the form of intravenous, intramuscular, intraperitoneal, or subcutaneous injections. Injection solutions are prepared by standard methods known in the art, for example as suspensions of the liposomes in sterile normal saline. It is also considered to apply the inventive single empty liposomes or the inventive mixture of empty liposomes as aerosol for the treatment of respiratory tract infections, or in a formulation useful for sublingual or buccal application, for intraocular or intravitreal application, as well as for topical application (e.g. eye drops, topical liquid suspensions for the skin, and the like). The preparation of such formulations from liposomes such as the empty liposomes of the invention is known in the art. Prophylactic measures based on the inventive single empty liposomes or the inventive mixture of empty liposomes will be helpful in the prevention of viral infections, for example in the prevention of diseases of the respiratory tract by the general population during seasonal influenza epidemics and in other settings that favour the spread of viral diseases.

In further embodiments, the method of treating or prevention a viral infection in accordance with the present invention further comprises the administration of a therapeutically effective amount of the inventive composition, the inventive single empty liposomes or the inventive mixture of empty liposomes to a mammal, preferably in a human in need thereof, wherein the inventive composition, the inventive single empty liposomes or the inventive mixture of empty liposomes are administered not in conjunction with any other drug, and thus typically alone, wherein further typically and preferably, the inventive composition, the inventive single empty liposomes or the inventive mixture of empty liposomes are not co-administered at the same time or any time of up to 4 weeks before and after, preferably up to 2 weeks before and after.

In their ability to inhibit a virus to bind to, fuse with, and infiltrate a target cell, the inventive single empty liposomes or the inventive mixture of empty liposomes are non-toxic and are unlikely to exert any selective antiviral pressure, which would further the emergence of drug-resistant viruses; they are an appealing alternative to other antiviral drugs.

In further embodiments of the present invention, the method further comprises the administration of a therapeutically effective amount of the inventive composition, the inventive single empty liposomes or the inventive mixture of empty liposomes to a mammal, preferably in a human in need thereof, before, after, together or in parallel with a standard antiviral treatment of the viral infection.

It is understood that the empty liposomes as defined above and the mixtures of empty liposomes as defined above may, if desired, be used together or in combination with further compounds. For example, it is possible to add components to prepare standard pharmaceutical compositions. It is also considered to add drugs or drug-like compounds, or to add further known or novel liposomes incorporating drugs or drug-like compounds in the liposome interior. Drugs and prodrugs considered are, in particular, standard viral treatments. Compounds considered are for example interferons, pegylated interferons, nucleoside inhibitors such as Ribavirin, protease inhibitors, polymerase inhibitors, nucleoside reverse transcriptase inhibitors (NRTIs), nonnucleoside reverse transcriptase inhibitors (NNRTIs), synthetic nucleoside analogues, entry inhibitors such as CCR5 co-receptor antagonists, HIV integrase strand transfer inhibitors, viral fusion inhibitors, neuraminidase inhibitors, viral protein inhibitors such as M2 channel inhibitors. Compounds considered are antiviral and antibacterial vaccines as well as antibacterial treatments.

Other drugs considered are for example anti-inflammatory drugs, including corticosteroids (glucocorticoids), such as hydrocortisone (cortisol), cortisone, prednisone, prednisolone, methylprednisolone, dexamethasone, betamethasone, triamcinolone, beclometasone, fludrocortisone acetate, deoxycorticosterone acetate (DOCA), aldosterone, budesonide, desonide, and fluocinonide; non-steroidal anti-inflammatory drugs, e.g. salicilates, such as aspirin (acetylsalicylic acid), diflunisal, and salsalate; propoinic acid derivatives, such as ibuprofen, dexibuprofen, naproxen, fenoprofen, ketoprofen, dexketoprofen, flurbiprofen, oxaprozin, and loxoprofen; acetic acid derivatives, such as indomethacin, tolmetin, sulindac, etodolac, ketorolac, diclofenac, and nabumetone; enolic acid (oxicam) derivatives, such as piroxicam, meloxicam, tenoxicam, droxicam, lornoxicam, and isoxicam; fenamic acid derivatives (fenamates), such as mefenamic acid, meclofenamic acid, flufenamic acid, and tolfenamic acid; selective COX-2 inhibitors (coxibs), such as Celecoxib; and others, such as licofelone.

Further drugs considered are for example vasopressors and vasoconstrictors, such as vasopressin, oxymetazoline, phenylephrine, propylhexedrine, pseudoephedrine, epinephrine, norepinephrine, dopamine, and antihistamines.

Also considered are other type of drugs, for example paracetamol (pain killer), amphotericin B (against fungal infections), bupivacaine (post-surgical pain control), morphine (pain killer), verteporfin (ophthalmological diseases), estradiol (menopausal disturbances), aganocide® compounds.

In this combination treatment the inventive empty liposomes and liposome mixtures may be considered as adjuvants, and the corresponding method of treatment as adjunct treatment.

### EXAMPLES

### Liposomes:

Sphingomyelin (SM; CAS No. 85187-10-6) from egg yolk was purchased from Sigma (S0756), Avanti Polar Lipids (860061) or Lipoid GmbH.
Cholesterol (CHOL; CAS No. 57-88-5) from ovine wool grease was purchased from Sigma (C-8667), Avanti Polar Lipids (70000) or Dishman Netherlands B.V.

It is in accordance with the present invention that sphingomyelin (SM) and cholesterol (CHOL) comprised by or consisted of by the inventive empty liposomes or the inventive mixtures of empty liposomes may be either obtained from natural sources as indicated above or alternatively by way of chemical synthesis.

### Liposomes preparation

Unilamellar sphingomyelin:cholesterol (35:65 molar ratio) and sphingomyelin only (100%) liposomes were prepared using sonication or microfluidization (e.g. high pressure homogenization or according to a hydration, extrusion, and diafiltration process protocol.

### Sonication:

The lipids were individually dissolved in chloroform at 1 mg/ml concentrations and stored at -20°C. For the preparation of liposomes the chloroform solutions of individual lipids were mixed in the proportions, as required, to produce routinely 50-500 µl of the final solution. Chloroform was completely evaporated for 20-50 min at 60°C. 50 µl or 100 µl of Tyrode's buffer (140 mM NaCl, 5 mM KCl, 1 mM MgCl₂, 10 mM glucose, 10 mM HEPES; pH=7.4) containing 2.5 mM CaCl₂ was added to the tubes containing films of dried lipids and vigorously vortexed. The lipid suspensions were incubated for 20-30 min at 45°C in an Eppendorf thermomixer with vigorous shaking. To produce liposomes, the final lipid suspensions were sonicated 3x5 sec at 6°C in a Bandelin Sonopuls sonicator at 70% power. The liposomal preparations were left for at least 1 hour at 6°C before they were used in experiments.

### Hydration, extrusion, and diafiltration process protocol:

In an alternative method, each liposomal formulation was made by the ethanol hydration and extrusion method. Lipids were individually dissolved in ethanol and *t*-butanol while mixing at elevated temperature (∼55°C). The lipid solution was then added to a PBS Buffer Solution (Sodium Chloride, Monosodium Phosphate, Dihydrate and Disodium Phosphate, Dihydrate dissolved in water for injection while mixing, adjusted to a pH of 7.0-7.4 with either hydrochloric acid (HCl) or sodium hydroxide (NaOH) as required, and filtered through a 0.2 µm filter) while mixing at elevated temperature (∼65°C) for approximately 30 minutes. The ensuing process fluid was then extruded repeatedly through a series of polycarbonate track-etched membranes at elevated pressure and temperature (∼65°C) until the desired particle size was achieved, as measured by dynamic light scattering (example of extruder: LIPEX® Extruders). The ensuing process fluid was then concentrated approximately 2-fold using a 100,000 molecular weight cut-off hollow-fiber cartridge and then diafiltered against approximately 10 volume exchanges of the PBS Buffer Solution to remove the ethanol and t-butanol. At the end of diafiltration, the process fluid was concentrated by approximately 30% to allow subsequent dilution to target lipid concentration. Before dilution, the process fluid was filtered through a 0.2 µm sterilizing-grade filter in order to remove any larger liposomes which could clog the filter during Sterile Filtration. The process fluid was then diluted to a target of 40 mg/mL total lipid with the PBS Buffer Solution. The final formulations were aseptically filtered through two 0.2 µm sterilizing grade filters in series and aseptically filled into glass tubing vials.

The concentration of individual lipids in the liposomes is always given as the weight per weight ratio. In liposomes containing sphingomyelin and cholesterol, the 1:1 (weight per weight) ratio corresponds to 50% (weight per weight) or to 35:65 molar ratio. Specifications are described in Table 1.

**Table 1: Specifications liposomes**

| Mean diameter (nm) | Poly-dispersity index | Zeta potential (mV) | Osmolality (mmol/kg) | pH |
|---|---|---|---|---|
| 40 to 400 | < 0.45 | -25 to +2 | 250-400 | 6.5-8.0 |

### EXAMPLE 1

### HCVpp Entry Inhibitor Assay

The HCV entry inhibitor assay used Huh 7.5 cells and HIV-1 virus which had been pseudotyped with the HCV (GT1a or GT1b) E1/E2. Briefly, cells were plated one day prior to assay. On the day of assay, media was aspirated and 50µl of 2X compound and 50µl of pretitered virus were added to each well. Toxicity plates received only the test compound in tissue culture medium. On day 5, the medium was removed from all wells of the efficacy plates, followed one wash with medium and addition of 100µl of firefly luciferase reagent and subsequent luciferase detection using a Microbeta detector (Wallac). For toxicity plates, 10µl of MTS reagent was added to all wells and incubated until cell control optical density values were between 1 and 2. Compound cytotoxicity was assessed by MTS (CellTiter®96 Reagent, Promega, Madison WI) dye reduction. The % reduction in viral entry was determined and reported; IC50 (concentration inhibiting virus entry by 50%), TC50 (concentration resulting in 50% cell death) and a calculated TI (therapeutic index TC50/ IC50) were provided along with a graphical representation of the antiviral activity and compound cytotoxicity when compounds were tested in dose-response. Each assay included 1µMg/ml cyanovirin and/or 10µg/ml antibody CD81 as a positive control.

### Cell Preparation

Huh 7.5 cells (human hepatocyte cell line) were obtained from APath LLC and were grown in Dulbecco's Modified Eagle's Medium (DMEM) supplemented with 5% fetal bovine serµM (FBS), 2.0 mM L-Glutamine, 100 units/ml Penicillin and 100 µg/ml Streptomycin, and 0.1 mM non-essential amino acids ("growth medium"). Cells were sub-cultured twice a week at a split ratio of 1:15 using standard cell culture techniques. Total cell number and percent viability determinations were performed using a hemacytometer and trypan blue exclusion. Cell viability must be greater than 95% for the cells to be utilized in the assay. The cells were seeded in 96-well tissue culture plates the day before the assay at a concentration of 8-10 x 103 cells/well.

### Virus Preparation

The virus used for this assay was HIV-1 where the HIV envelope was knocked out and then pseudotyped with the HCV (GT1a or GT1b) E1/E2. This virus also had a luciferase reporter gene. For each assay, a pre-titered aliquot of virus was removed from the freezer (-80oC) and allowed to thaw slowly to room temperature in a biological safety cabinet. The virus was resuspended and diluted into tissue culture medium such that the amount of virus added to each well was at MOI (multiplicity of infection) of 1.

### Plate Format

Each efficacy plate contained cell control wells (cells only), virus control wells (cells plus virus), and experimental wells (drug plus cells plus virus). Toxicity plate wells contained cell control wells (cells only) and experimental wells (cells plus drug in the absence of virus). Samples were evaluated for antiviral efficacy with triplicate measurements using 6 concentrations at halflog (or other) dilutions (12 concentrations could also be performed) in order to determine IC50 values and cellular cytotoxicity, if detectable.

Table 2 and Table 3 represent the standard efficacy and toxicity plate formats, respectively, for testing compounds at 6 concentrations using a representative high-test concentration of 100 µM.

**Table 2: Efficacy plate format**

| | 1 | 2 | 3 | 4 | 5 | 6 | 7 | 8 | 9 | 10 | 11 | 12 |
|---|---|---|---|---|---|---|---|---|---|---|---|---|
| A | CELL CONTROL (CELLS ONLY) | | | | | | | | | | | |
| B | Cells+Virus + Drug 1 100µM | | | Cells+Virus + Drug 2 100µM | | | Cells+Virus + Drug 3 100µM | | | Cells+Virus + Drug 4 100µM | | |
| C | Cells+Virus + Drug 1 32µM | | | Cells+Virus + Drug 2 32µM | | | Cells+Virus + Drug 3 32µM | | | Cells+Virus + Drug 4 32µM | | |
| D | Cells+Virus + Drug 1 10µM | | | Cells+Virus + Drug 2 10µM | | | Cells+Virus + Drug 3 10µM | | | Cells+Virus + Drug 4 10µM | | |
| E | Cells+Virus + Drug 1 3.2µM | | | Cells+Virus + Drug 2 3.2µM | | | Cells+Virus + Drug 3 3.2µM | | | Cells+Virus + Drug 4 3.2µM | | |
| F | Cells+Virus + Drug 11µM | | | Cells+Virus + Drug 2 1µM | | | Cells+Virus + Drug 3 1µM | | | Cells+Virus + Drug 4 1µM | | |
| G | Cells+Virus + Drug 1 0.32µM | | | Cells+Virus + Drug 2 0.32µM | | | Cells+Virus + Drug 3 0.32µM | | | Cells+Virus + Drug 4 0.32µM | | |
| H | VIRUS CONTROL (CELLS+VIRUS) | | | | | | | | | | | |

**Table 3: Toxicity plate format**

| | 1 | 2 | 3 | 4 | 5 | 6 | 7 | 8 | 9 | 10 | 11 | 12 |
|---|---|---|---|---|---|---|---|---|---|---|---|---|
| A | CELL CONTROL (CELLS ONLY) | | | | | | | | | | | |
| B | Cells + Drug 1 100µM | | | Cells + Drug 2 100µM | | | Cells + Drug 3 100µM | | | Cells + Drug 4 100µM | | |
| C | Cells + Drug 1 32µM | | | Cells + Drug 2 32µM | | | Cells + Drug 3 32µM | | | Cells + Drug 4 32µM | | |
| D | Cells + Drug 1 10µM | | | Cells + Drug 2 10µM | | | Cells + Drug 3 10µM | | | Cells + Drug 4 10µM | | |
| E | Cells + Drug 1 3.2µM | | | Cells + Drug 2 3.2µM | | | Cells + Drug 3 3.2µM | | | Cells + Drug 4 3.2µM | | |
| F | Cells + Drug 1 1µM | | | Cells + Drug 2 1 µM | | | Cells + Drug 3 1µM | | | Cells + Drug 4 1 µM | | |
| G | Cells + Drug 1 0.32µM | | | Cells + Drug 2 0.32µM | | | Cells + Drug 3 0.32µM | | | Cells + Drug 4 0.32µM | | |
| H | CELL CONTROL (CELLS ONLY) | | | | | | | | | | | |

### EXAMPLE 2

### Inhibition of HCV-lb by the inventive mixture of empty liposomes

A very preferred inventive mixture of empty liposomes displayed antiviral efficacy. Said preferred inventive mixture of empty liposomes consists of a 1:1 (weight per weight) mixture of said first empty liposomes and said second liposomes, wherein said first empty liposomes is composed of a 1:1 weight ratio (1:1 weight per weight; 35:65 molar ratio) of sphingomyelin (SM) and cholesterol (CHOL), and said second empty liposomes is composed exclusively of SM. Notably, said very preferred inventive mixture of empty liposomes inhibited HCV infection in a dose-dependent manner without apparent cytotoxicity. This positive antiviral efficacy using pseudotyped HCV particles indicates that the inventive mixture of empty liposomes inhibited the entry step in the HCV life cycle (FIG. 1).

FIG. 1 shows dose dependent inhibition of entry step into Huh7.5 cells by said inventive mixture of empty liposomes. Huh7.5 cells were infected with HCVpp in the presence of various concentrations of inventive mixture of empty liposomes. Luciferase activity was measured using a Microbeta detector, in order to determine IC50 values (concentration inhibiting virus entry by 50%). Data are presented in FIG.1 as percent inhibition relative to control infections lacking compound (luciferase-positive Huh7.5 cells not treated with inventive mixture of empty liposomes) (Percent of Virus Control: %VC).

Results are expressed as mean ± standard deviation of triplicate experiments. The inventive mixture of empty liposomes displays no apparent cytotoxicity. Huh7.5 cells were infected with HCVpp in the presence of said inventive mixture of empty liposomes, using 6 dilutions. The cytotoxicity of said inventive mixture of empty liposomes was assessed by MTS dye reduction in order to determine TC50 values (concentration resulting in 50% cell death), and was compared to that of Huh7.5 cells not treated with said inventive mixture of empty liposomes (Percent of Cell Control: %CC). A therapeutic index (TI = TC₅₀/ IC₅₀) was therefore calculated.

The following values were obtained: IC₅₀: 49.7 ug/ml; IC₉₀: >2000 ug/ml; TC₅₀: >2000 ug/ml and TI: >40.24.

Each assay included 1µMg/ml cyanovirin (PG51) (FIG. 2) and/or 10µg/ml antibody CD81 (FIG. 3) as a positive control for reducing HCV entry to the cells. The respective values were obtained: IC₅₀: 40.5 ng/ml (for cyanovirin (P51G)) and 221.5 ug/ml (for anti-CD81); IC₉₀: 208.9 ng/ml (for cyanovirin (P51G)) and 1196.8 ng/ml (for anti-CD81); TC₅₀: >1000 ng/ml (for cyanovirin (P51G)) and >5000 ng/ml (for anti-CD81), and TI: >24.69 (for cyanovirin (P51G)) and >22.57 (for anti-CD81).

This study demonstrates, for the first time, that lipid raft-mimicking liposomal agents, exclusively composed of phospholipids, are sufficient to efficaciously prevent HCV viral entry. Inhibition of viral fusion and/or entry has a high preventative and therapeutic potential. Therapeutic approaches targeting viral fusion and/or entry mainly aim at modulating the virus envelope with a view to preventing host cell binding and thus acting as viral fusion / entry blockers. In contrast, the preferred inventive mixture of empty liposomes acts as a decoy to which the viral envelope preferentially binds instead of targeting host cells. Therefore, it consists of an anti-viral compound with a new mode of action.

### EXAMPLE 3

### HCVcc Entry Inhibitor Assay

The preferred inventive mixture of empty liposomes of Example 2 is assessed using the HCVcc system. More precisely, Huh-7 hepatoma cells are infected with HCVcc genotype 2a chimeric virus (HCV2aCh) bearing or not a Renilla luciferase reporter gene, in the presence of various concentrations of inventive mixture of empty liposomes. HCVcc infection levels are evaluated by measuring luciferase activity or using quantitative reverse transcription polymerase chain reaction (qRT-PCR), in order to determine IC50 values (concentration inhibiting virus entry by 50%). A therapeutic index is determined based on the lethal dose over effective dose.

### EXAMPLE 4

### HCV Replicon Combination Antiviral Assay

The preferred inventive mixture of empty liposomes of Example 2 is assessed using a Huh7 human hepatoma cell line that contains an HCV genotype 1b (Con1 strain) subgenomic replicon with a stable luciferase reporter and three cell culture-adaptive mutations, the ET cell line. Luciferase activity is measured in order to assess the impact on HCV replication, alone or in combinations with other drug for HCV treatment and HCV drug candidates. Resistance / cross-resistance selection tests are included.

### EXAMPLE 5

### In vivo Animal Models

The preferred inventive mixture of empty liposomes of Example 2 is assessed in animal models of viral infections. Single or multiple administrations of said inventive mixture of empty liposomes, at different time points in the development of the disease, are carried out via intravenous or subcutaneous or intranasal or intracolonic route.

For the evaluation of the protective effects of said inventive mixture of empty liposomes against HCV, said inventive mixture of empty liposomes is administered to a human-liver chimeric mouse model, entry factor immunocompetent transgenic mouse model or humanized mice infected with HCV. Said inventive mixture of empty liposomes is given alone or in combination with another antiviral treatment, such as nucleoside/nucleotide and nonnucleoside polymerase inhibitors, nonstructural protein 5A (NS5A) inhibitors, protease inhibitors, microRNA-targeting agents, a scavenger receptor B1 antagonist, or interferon-alpha. The therapeutic potential is evaluated by assessing liver disease, infection persistency, and immune response.

For the evaluation of the protective effects of said inventive mixture of empty liposomes against HIV, humanized mouse models are treated with said inventive mixture of empty liposomes.

### EXAMPLE 6

### HIV CXCR4-Tropic In Vitro Assay

The preferred inventive mixture of empty liposomes of Example 2 is incubated with HeLa CD4 LTR β-gal for 1h at 37°C; HL2/3 cells is then added. 48h after HL2/3 cells addition, β-galactosidase enzyme expression is determined by chemiluminescence. The HL2/3 cell line, which has a high production of HIV-1 Env, Tat, Rev, and Nef proteins, triggers CD4 dependent HIV-1 Env mediated cell fusion which leads to cytoplasmic mixing of the contents of the two cell lines and subsequent β-galactosidase expression. Blocking fusion leads to a decrease in β-galactosidase expression levels. As described in Example 1, the cytotoxicity of the inventive mixture of empty liposomes is assessed in parallel using MTS dye reduction.

### EXAMPLE 7

### HIV CCR5-Tropic In Vitro Assay

The preferred inventive mixture of empty liposomes of Example 2 is incubated with MAGI-R5 and HeLa R5-16 cells for 40 to 48h. MAGI-R5 cells and HeLa R5-16 cells co-incubation causes cell fusion which leads to cytoplasmic mixing of the contents of the two cell lines and subsequent β-galactosidase expression. β-galactosidase enzyme expression is determined by chemiluminescence. Blocking fusion leads to a decrease in β-galactosidase expression levels.

### EXAMPLE 8

### In Vitro tests against HSV-1

Confluent Vero cell monolayers are infected with different dilutions of HSV-1 and exposed to the preferred inventive mixture of empty liposomes of Example 2. Cytopathic effects are observed and plaques visualized, for example by staining cells with crystal violet, and counted. Acyclovir is used as positive control.

### EXAMPLE 9

### In Vitro tests against Influenza

Viral-induced cytopathic effects in the presence or absence of the preferred inventive mixture of empty liposomes of Example 2 are assessed using adenocarcinomic human alveolar cells (A549) and Madin-Darby canine kidney cells (MDCK) exposed to Influenza. Ribavirin is used as positive control.

### EXAMPLE 10

### In Vitro tests against Measles virus

Viral-induced cytopathic effects in the presence or absence of the preferred inventive mixture of empty liposomes of Example 2 are assessed using human diploid cell MRC-5 exposed to Measles virus. Protective effects of said inventive mixture of empty liposomes are evaluated alone or in combination to current inhibitor drugs, such as neuraminidase and M2, with a view to assess its potential to help prevent and control pandemics of influenza.

## Claims

1. A composition comprising, preferably consisting of,
(i) a single empty liposome, wherein said single empty liposome is selected from
(a) an empty liposome consisting of sphingomyelin and cholesterol, wherein the amount of cholesterol is at least 20% (weight per weight); or
(b) an empty liposome consisting of sphingomyelin;
or
(ii) a mixture of empty liposomes; wherein said mixture of empty liposomes comprises
(a) a first empty liposome consisting of sphingomyelin and cholesterol, wherein the amount of cholesterol is at least 20% (weight per weight); and
(b) a second empty liposome consisting of sphingomyelin;
for use in a method of treating or preventing a viral infection in a mammal, preferably in a human.

2. The composition for use of claim 1, wherein said composition comprises, preferably consists of, a single empty liposome, wherein said single empty liposome is an empty liposome consisting of sphingomyelin and cholesterol, wherein the amount of cholesterol is at least 20% (weight per weight).

3. The composition for use of claim 1, wherein said composition comprises, preferably consists of, a mixture of empty liposomes, wherein said mixture of empty liposomes comprises (a) a first empty liposome consisting of sphingomyelin and cholesterol, wherein the amount of cholesterol is at least 20% (weight per weight); and (b) a second empty liposome consisting of sphingomyelin.

4. The composition for use of any one of the claims 1 to 3, wherein the amount of cholesterol of said empty liposome is 20%-70% (weight per weight), and wherein preferably the amount of cholesterol of said empty liposome is 25%-60% (weight per weight).

5. The composition for use of any one of the claims 1 to 4, wherein the amount of cholesterol of said empty liposome is 45%-55% (weight per weight), and wherein preferably the amount of cholesterol of said empty liposome is about 50% (weight per weight).

6. The composition for use of any one of the claims 1 to 5, wherein said mixture of empty liposomes comprises at least 30% (weight per weight) of said first empty liposome and at most 70% (weight per weight) of said second empty liposome, and wherein preferably said mixture of empty liposomes comprises at least 40% (weight per weight) of said first empty liposome and at most 60% (weight per weight) of said second empty liposome.

7. The composition for use of any one of the claims 1 to 6, wherein said mixture of empty liposomes comprises at least 45% (weight per weight) of said first empty liposome and at most 55% (weight per weight) of said second empty liposome, and wherein preferably said mixture of empty liposomes comprises about 50% (weight per weight) of said first empty liposome and about 50% (weight per weight) of said second empty liposome.

8. The composition for use of claim 1, wherein said composition comprises, preferably consists of, a single empty liposome, wherein said single empty liposome is an empty liposome consisting of sphingomyelin.

9. The composition for use of any one of the claims 1 to 8, wherein said viral infection is an infection by an enveloped virus.

10. The composition for use of claim 9, wherein said enveloped virus is selected from a hepatitis C virus (HCV), an immunodeficiency virus (HIV), a T lymphotrophic virus, a herpesvirus, a measles virus, a chicken pox virus, an influenza virus, an Ebola virus or a Marburg virus.

11. The composition for use of any one of the claims 1 to 8, wherein said viral infection is a hepatitis C virus infection.

12. The composition for use of any one of the claims 1 to 8, wherein said viral infection is an immunodeficiency virus (HIV) infection, and wherein preferably said viral infection is an immunodeficiency virus type-1 (HIV-1) infection.

13. The composition for use of any one of the claims 1 to 8, wherein said viral infection is a herpesvirus infection, wherein preferably said herpesvirus infection is a herpes simplex virus type-1 (HSV-1) infection or a human herpesvirus 6 (HHV6) infection.

14. The composition for use of any one of the claims 1 to 8, wherein said viral infection is an influenza virus infection or a measles virus infection.

15. The composition for use of any one of the claims 1 to 8, wherein said viral infection is an Ebola virus infection or a Marburg virus infection.

## Patentansprüche

1. Eine Zusammensetzung, umfassend, vorzugsweise bestehend aus:
(i) einem einzelnen leeren Liposom, wobei das einzelne leere Liposom ausgewählt ist aus:
(a) einem leeren Liposom, bestehend aus Sphingomyelin und Cholesterin, wobei die Cholesterinmenge mindestens 20 % beträgt (Gewichtsanteil); oder
(b) einem leeren Liposom bestehend aus Sphingomyelin;
oder
(ii) einer Mischung aus leeren Liposomen, wobei die Mischung aus leeren Liposomen Folgendes umfasst:
(a) ein erstes leeres Liposom, bestehend aus Sphingomyelin und Cholesterin, wobei die Cholesterinmenge mindestens 20 % (Gewichtsanteil) beträgt; und
(b) ein zweites leeres Liposom, bestehend aus Sphingomyelin;
zur Verwendung in einem Verfahren zur Behandlung oder Verhinderung einer Virusinfektion bei einem Säugetier, vorzugsweise bei einem Menschen.

2. Zusammensetzung zur Verwendung nach Anspruch 1, wobei die Zusammensetzung ein einzelnes leeres Liposom umfasst oder vorzugsweise daraus besteht, wobei das einzelne leere Liposom ein leeres Liposom ist, das aus Sphingomyelin und Cholesterin besteht, wobei die Menge an Cholesterin mindestens 20 % (Gewichtsanteil) beträgt.

3. Zusammensetzung zur Verwendung nach Anspruch 1, wobei die Zusammensetzung eine Mischung leerer Liposomen umfasst oder vorzugsweise daraus besteht, wobei die Mischung leerer Liposomen (a) ein erstes leeres Liposom umfasst, das aus Sphingomyelin und Cholesterin besteht, wobei die Menge an Cholesterin bei mindestens 20 % (Gewichtsanteil) liegt, und (b) ein zweites leeres Liposom, das aus Sphingomyelin besteht.

4. Zusammensetzung zur Verwendung nach einem der Ansprüche 1 bis 3, wobei die Cholesterinmenge des leeren Liposoms 20 bis 70 % (Gewichtsanteil) beträgt und wobei die Cholesterinmenge des leeren Liposoms vorzugsweise 25 % bis 60 % (Gewichtsanteil) beträgt.

5. Zusammensetzung zur Verwendung nach einem der Ansprüche 1 bis 4, wobei die Cholesterinmenge des leeren Liposoms 45 bis 55 % (Gewichtsanteil) beträgt und wobei die Cholesterinmenge des leeren Liposoms vorzugsweise etwa 50 % beträgt (Gewichtsanteil).

6. Zusammensetzung zur Verwendung nach einem der Ansprüche 1 bis 5, wobei die Mischung leerer Liposomen mindestens 30 % (Gewichtsanteil) des ersten leeren Liposoms und höchstens 70 % (Gewichtsanteil) des zweiten leeren Liposoms umfasst und wobei vorzugsweise die Mischung aus leeren Liposomen mindestens 40 % (Gewichtsanteil) des ersten leeren Liposoms und höchstens 60 % (Gewichtsanteil) des zweiten leeren Liposoms umfasst.

7. Zusammensetzung zur Verwendung nach einem der Ansprüche 1 bis 6, wobei die Mischung leerer Liposomen mindestens 45 % (Gewichtsanteil) des ersten leeren Liposoms und höchstens 55 % (Gewichtsanteil) des zweiten leeren Liposoms umfasst und wobei vorzugsweise die Mischung aus leeren Liposomen etwa 50 % (Gewichtsanteil) des ersten leeren Liposoms und etwa 50 % (Gewichtsanteil) des zweiten leeren Liposoms umfasst.

8. Zusammensetzung zur Verwendung nach Anspruch 1, wobei die Zusammensetzung ein einzelnes leeres Liposom umfasst oder vorzugsweise daraus besteht, wobei das einzelne leere Liposom ein leeres Liposom ist, das aus Sphingomyelin besteht.

9. Zusammensetzung zur Verwendung nach einem der Ansprüche 1 bis 8, wobei die Virusinfektion eine Infektion durch ein umhülltes Virus ist.

10. Zusammensetzung zur Verwendung nach Anspruch 9, wobei das umhüllte Virus ausgewählt ist aus einem Hepatitis-C-Virus (HCV), einem Immundefizienzvirus (HIV), einem T-lymphotrophen Virus, einem Herpesvirus, einem Masernvirus, einem Windpockenvirus, einem Influenzavirus, einem Ebola-Virus oder einem Marburg-Virus.

11. Zusammensetzung zur Verwendung nach einem der Ansprüche 1 bis 8, wobei die Virusinfektion eine Infektion mit dem Hepatitis-C-Virus ist.

12. Zusammensetzung zur Verwendung nach einem der Ansprüche 1 bis 8, wobei die Virusinfektion eine Infektion mit dem Immundefizienzvirus (HIV) ist und wobei die Virusinfektion vorzugsweise eine Infektion mit dem Immundefizienzvirus Typ 1 (HIV-1) ist.

13. Zusammensetzung zur Verwendung nach einem der Ansprüche 1 bis 8, wobei die Virusinfektion eine Infektion mit dem Herpesvirus ist, wobei die Infektion mit dem Herpesvirus vorzugsweise eine Infektion mit dem Herpes-simplex-Virus Typ 1 (HSV-1) oder eine Infektion mit einem humanen Herpesvirus 6 (HHV6) ist.

14. Zusammensetzung zur Verwendung nach einem der Ansprüche 1 bis 8, wobei die Virusinfektion eine Infektion mit dem Influenzavirus oder eine Infektion mit dem Masernvirus ist.

15. Zusammensetzung zur Verwendung nach einem der Ansprüche 1 bis 8, wobei die Virusinfektion eine Infektion mit dem Ebola-Virus oder eine Infektion mit dem Marburg-Virus ist.

## Revendications

1. Composition comprenant, et de préférence constituée,
(i) d'un seul liposome vide, dans laquelle ledit seul liposome vide est choisi parmi
(a) un liposome vide constitué de sphingomyéline et de cholestérol, dans laquelle la quantité de cholestérol est d'au moins 20 % (poids par poids) ; ou
(b) d'un liposome vide composé de sphingomyéline ;
ou
(ii) d'un mélange de liposomes vides ; dans laquelle ledit mélange de liposomes vides comprend
(a) un premier liposome vide constitué de sphingomyéline et de cholestérol, dans laquelle la quantité de cholestérol est d'au moins 20 % (poids par poids) ; et
(b) un second liposome vide constitué de sphingomyéline ;
pour une utilisation dans une méthode de traitement ou de prévention d'une infection virale chez un mammifère, de préférence chez l'homme.

2. Composition pour une utilisation selon la revendication 1, dans laquelle ladite composition comprend, et est de préférence constituée, d'un seul liposome vide, dans laquelle ledit seul liposome vide est un liposome vide constitué de sphingomyéline et de cholestérol, dans laquelle la quantité de cholestérol est d'au moins 20 % (poids par poids).

3. Composition pour une utilisation selon la revendication 1, dans laquelle ladite composition comprend, et est de préférence constituée, d'un mélange de liposomes vides, dans laquelle ledit mélange de liposomes vides comprend (a) un premier liposome vide constitué de sphingomyéline et de cholestérol, dans laquelle la quantité de cholestérol est d'au moins 20 % (poids par poids) ; et (b) un second liposome vide constitué de sphingomyéline.

4. Composition pour une utilisation selon l'une quelconque des revendications 1 à 3, dans laquelle la quantité de cholestérol dudit liposome vide est de 20 % à 70 % (poids par poids), et dans laquelle la quantité de cholestérol dudit liposome vide est de préférence de 25 % à 60 % (poids par poids).

5. Composition pour une utilisation selon l'une quelconque des revendications 1 à 4, dans laquelle la quantité de cholestérol dudit liposome vide est de 45 % à 55 % (poids par poids), et dans laquelle de préférence la quantité de cholestérol dudit liposome vide est d'environ 50 % (poids par poids).

6. Composition pour une utilisation selon l'une quelconque des revendications 1 à 5, dans laquelle ledit mélange de liposomes vides comprend au moins 30 % (poids par poids) dudit premier liposome vide et au plus 70 % (poids par poids) dudit second liposome vide, et dans lequel ledit mélange de liposomes vides comprend de préférence au moins 40 % (poids par poids) dudit premier liposome vide et au plus 60 % (poids par poids) dudit second liposome vide.

7. Composition pour une utilisation selon l'une quelconque des revendications 1 à 6, dans laquelle ledit mélange de liposomes vides comprend au moins 45 % (poids par poids) dudit premier liposome vide et au plus 55 % (poids par poids) dudit second liposome vide, et dans laquelle ledit mélange de liposomes vides comprend de préférence environ 50 % (poids par poids) dudit premier liposome vide et environ 50 % (poids par poids) dudit second liposome vide.

8. Composition pour une utilisation selon la revendication 1, dans laquelle ladite composition comprend, et est de préférence constituée, d'un seul liposome vide, dans laquelle ledit seul liposome vide est un liposome vide constitué de sphingomyéline.

9. Composition pour une utilisation selon l'une quelconque des revendications 1 à 8, dans laquelle ladite infection virale est une infection par un virus enveloppé.

10. Composition pour une utilisation selon la revendication 9, dans laquelle ledit virus enveloppé est choisi parmi un virus de l'hépatite C (VHC), un virus d'immunodéficience (VIH), un virus T-lymphotrophique, un herpèsvirus, un virus de la rougeole, un virus de la varicelle, un virus de la grippe aviaire, un virus Ebola ou un virus Marburg.

11. Composition pour une utilisation selon l'une quelconque des revendications 1 à 8, dans laquelle ladite infection virale est une infection par le virus de l'hépatite C.

12. Composition pour utilisation selon l'une quelconque des revendications 1 à 8, dans laquelle ladite infection virale est une infection par le virus d'immunodéficience (VIH), et dans laquelle de préférence ladite infection virale est une infection par le virus d'immunodéficience de type 1 (VIH-1).

13. Composition pour une utilisation selon l'une quelconque des revendications 1 à 8, dans laquelle ladite infection virale est une infection à l'herpèsvirus, dans laquelle ladite infection à l'herpèsvirus est de préférence une infection par le virus de l'herpès simplex de type 1 (HSV-1) ou une infection par l'herpèsvirus humain 6 (HHV6).

14. Composition pour une utilisation selon l'une quelconque des revendications 1 à 8, dans laquelle ladite infection virale est une infection par le virus de la grippe ou une infection par le virus de la rougeole.

15. Composition pour une utilisation selon l'une quelconque des revendications 1 à 8, dans laquelle ladite infection virale est une infection par le virus Ebola ou une infection par le virus de Marburg.
